# DEMANDE DE BREVET EUROPEEN

(11) **EP 4 494 630 A1**
(43) Date de publication de la demande: **22.01.2025**
(21) Numéro de dépôt: 23187073.4
(22) Date de dépôt: 21.07.2023
(51) Int. Cl.: A61K 8/60, A61K 8/97, A61Q 19/00, A61Q 19/08, A61P 17/00, A61P 29/00

(54) **EXTRAIT ALCOOLIQUE DE PARTIES AERIENNES FLEURIES DE TANAISIE, TANACETUM VULGARE, SON PROCEDE D'OBTENTION, ET COMPOSITION COSMETIQUE LE CONTENANT**

(71) Demandeur: Chanel Parfums Beauté, 92200 Neuilly-sur-Seine (FR)
(72) Inventeur: COCANDEAU, Vincent, 93694 PANTIN CEDEX (FR); LERISSON, Annie, 93694 PANTIN CEDEX (FR)
(74) Mandataire: Plasseraud IP

(57) **Abrégé**

L'invention concerne un extrait de parties aériennes fleuries de Tanaisie, *Tanacetum vulgare* son procédé d'obtention, et composition cosmétique le contenant, ainsi que différentes utilisations cosmétiques. Il présente des propriétés cosmétiques anti-âge, similaires aux propriétés du rétinol sans les inconvénients du rétinol. Il permet en effet une augmentation significative de l'épaisseur de l'épiderme, le renforcement de la fonction barrière en augmentant la synthèse de la Loricrine et permet d'augmenter le renouvellement tissulaire. Il a aussi démontré qu'il permet de réduire la propagation de la sénescence cutanée.

## Description

L'invention concerne un extrait des parties aériennes fleuries de Tanaisie, *Tanacetum vulgare*, son procédé d'obtention, une composition cosmétique le contenant, ainsi que différentes utilisations cosmétiques.

La peau est principalement constituée de trois couches, à savoir, en partant de la plus superficielle, l'épiderme, le derme et l'hypoderme.

L'épiderme contribue largement à assurer la protection de la peau et à en maintenir son bon fonctionnement et elle est la première barrière de protection de l'organisme vis-à-vis de l'environnement.

De ce fait, elle subit donc de nombreuses agressions extérieures qui peuvent conduire à des réactions cutanées d'inconfort voire, en cas de réactions très intenses ou plus graves, à des phénomènes d'irritation et/ou d'inflammation cutanées.

Les réactions cutanées d'inconfort, voire alternativement des réactions d'irritation et/ou d'inflammation de la peau peuvent notamment être induites par le contact avec des produits chimiques tels que les nettoyants, ou provenir d'actions mécaniques tels que le rasage, le gommage, le peeling, l'épilation.

Il existe toujours un besoin pour de nouveaux agents apaisants dans le domaine cosmétique. Il existe également un besoin pour de nouveaux agents, aptes à traiter et/ou diminuer les réactions d'irritation et/ou d'inflammation cutanées dans le domaine dermatologique.

Le derme est un tissu conjonctif assurant à la fois les fonctions de cohésion et de nutrition de la peau.

Le vieillissement cutané résulte de deux processus distincts et indépendants qui font intervenir des facteurs intrinsèques ou extrinsèques.

Le vieillissement intrinsèque ou chronobiologique correspond au vieillissement « normal » ou physiologique lié à l'âge.

Le vieillissement extrinsèque correspond au vieillissement provoqué d'une manière générale par l'environnement et plus particulièrement au photovieillissement dû à l'exposition au soleil.

La présente invention s'intéresse au vieillissement cutané intrinsèque ou physiologique ainsi qu'au vieillissement cutané extrinsèque.

Le vieillissement cutané est consécutif à une transformation des tissus conjonctifs et à l'abaissement de la capacité de régénération cellulaire. Cet effet se manifeste par l'apparition de ridules et de stigmates au cours du temps. La microcirculation diminue au niveau du derme superficiel. Les macromolécules telles que le collagène, l'élastine et les glycosaminoglycanes, dont l'acide hyaluronique est l'un des constituants, sont chimiquement modifiées. L'épaisseur même du derme régresse, les fibres sont dégradées et la peau perd ses propriétés biomécaniques et élastiques. Les phénomènes d'oxydation chimique et enzymatique augmentent avec l'âge et entraîne l'accroissement des réactions de pontage entre les fibres telles que les fibres de collagènes.

Les altérations associées au vieillissement peuvent se manifester de différentes manières, parmi lesquelles on peut citer :
- une désorganisation des fibres d'élastine entraînant une perte de fermeté, de souplesse et d'élasticité ou par l'apparition de télangiectasies;
- la perte d'éclat due à la réduction de la microcirculation et à un ralentissement du renouvellement cellulaire au niveau de l'épiderme et l'apparition de fines rides ou ridules ;
- le jaunissement de la peau qui développe un aspect parcheminé accompagné de l'apparition de taches pigmentaires associées à un dysfonctionnement de la synthèse de la mélanine (ou mélanogénèse) ;
- la sécheresse cutanée résultant d'une diminution de la fonction barrière de la couche cornée et à un ralentissement du renouvellement épidermique.

Il existe un besoin de fournir un agent actif polyfonctionnel susceptible d'agir sur un ensemble de causes d'altérations de la peau dues au vieillissement et/ou à une modification des mécanismes physiologiques liés au vieillissement ou apparentés.

Or, la Demanderesse a maintenant trouvé qu'un extrait alcoolique de parties aériennes fleuries de Tanaisie, *Tanacetum vulgare*, obtenu par un procédé particulier, présente, par le biais de stimulation ou d'inhibition de mécanismes physiologiques, des activités intéressantes vis-à-vis du vieillissement cutané et la réduction de l'inflammation.

En effet, comme démontré en exemples, l'extrait alcoolique de parties aériennes fleuries de Tanaisie, *Tanacetum vulgare*, selon l'invention présente des propriétés cosmétiques intéressantes : il permet une stimulation significative des voies biologiques en lien avec le vieillissement cutané et l'expression de CCL2 et CCL5, des molécules faisant partie du SASP5, dans des fibroblastes sénescents. Il présente des propriétés similaires à celle du rétinol sans les inconvénients du rétinol. Il permet en effet une augmentation significative de l'épaisseur de l'épiderme, le renforcement de la fonction barrière en augmentant la synthèse de la Loricrine et permet d'augmenter le renouvellement tissulaire.

D'autre part, l'extrait présente des propriétés cosmétiques apaisantes car il inhibe les voies biologiques de l'inflammation cutanée et celle des interférons, des cytokines immunomodulatrices connues pour leur rôle régulateur dans l'inflammation cutanée.

L'invention concerne donc, selon un premier aspect, un extrait alcoolique de parties aériennes fleuries de Tanaisie, *Tanacetum vulgare*, comprenant au moins des sucres, des polysaccharides et des dérivés caféiques.

L'invention a également pour objet, selon un second aspect, un procédé d'extraction de parties aériennes fleuries de Tanaisie, *Tanacetum vulgare*, comprenant au moins les étapes suivantes :
a) le broyage des parties aériennes fleuries de Tanaisie, Tanacetum vulgare,
b) au moins une, de préférence au moins deux extractions des dites parties aériennes fleuries broyées en présence d'un premier solvant alcoolique, chaque extraction étant suivie d'une étape de filtration ;
c) le mélange des filtrats liquides issus des extractions de l'étape b) ;
d) filtration du mélange obtenu en c) et, optionnellement, décoloration du filtrat obtenu par adoption sur charbon actif;
e) dilution dans un deuxième solvant alcoolique autre que ledit premier solvant alcoolique du mélange obtenu en d), et élimination du premier solvant alcoolique.

L'invention se rapporte également à un extrait alcoolique de parties aériennes fleuries de Tanaisie, *Tanacetum vulgare* susceptible d'être obtenu par un tel procédé.

L'invention se rapporte aussi à une utilisation cosmétique d'un extrait alcoolique de parties aériennes fleuries de Tanaisie, *Tanacetum vulgare* obtenu par un tel procédé, pour le renforcement de la fonction barrière et d'hydratation ou comme agent apaisant.

L'invention se rapporte en outre aussi à une composition cosmétique comprenant, dans un véhicule cosmétiquement acceptable, un extrait alcoolique de parties aériennes fleuries de Tanaisie, *Tanacetum vulgare*, selon le premier aspect. Par véhicule cosmétiquement acceptable, on entend un milieu compatible avec la peau, les muqueuses et les phanères. De préférence, la composition cosmétique selon l'invention est adaptée à une application par voie topique.

### Figures

Les Figures 1A et AB illustrent la diminution de l'expression de CCL2 (qPCR) et du relargage de CCL5 (dosage) après traitement de fibroblastes sénescents avec l'extrait de Tanaisie selon l'invention.
La Figure 2 illustre l'augmentation de l'épaisseur de l'épiderme après traitement des explants par l'extrait de Tanaisie selon l'invention.
La Figure 3 illustre le renforcement de la fonction barrière des explants traités par l'extrait de Tanaisie selon l'invention.
La Figure 4 illustre le renouvellement tissulaire renforcé par action de l'extrait de Tanaisie selon l'invention.

La matière première mise en oeuvre est constituée de parties aériennes fleuries de Tanaisie, *Tanacetum vulgare*.

Tanaisie, appelée aussi Barbotine, Herbe amère, Herbe de Saint Marc, Sent-bon, tanacée est une plante appartenant à famille *Asteraceae.* C'est une plante aromatique, vivace et herbacée, atteignant 1 m à un rythme de croissance rapide.

Les feuilles sont alternées et profondément divisées et les fleurs sont jaunes, nombreuses et en forme de boutons, se présentant en grappes denses, d'août à septembre.

Elle est originaire d'Europe et du nord de l'Asie et naturalisé en Amérique du Nord et du Sud.

C'est une espèce que pousse jusqu'à 1500m d'altitude, sur les prairies, dans les fourrés de buissons, sur les clairières et les lisières et préfère un sol sablonneux, limono-sableux et bien drainé, et le plein soleil.

Les parties aériennes fleuries de Tanaisie, *Tanacetum vulgare* utilisées selon l'invention sont typiquement choisies parmi les fleurs, les tiges et leurs mélanges. De préférence, les parties aériennes fleuries utilisées sont un mélange de fleurs et tiges de Tanaisie, *Tanacetum vulgare.* De préférence, ces parties aériennes fleuries sont préalablement séchées, puis broyées ou réduites en morceaux de manière usuelle, pour, de préférence, se présenter sous la forme d'une poudre de taille inférieure à 2 cm.

L'extrait sec de parties aériennes fleuries de Tanaisie, *Tanacetum vulgare* objet de la présente invention est composé de plusieurs familles de métabolites primaires et secondaires d'intérêt.

Parmi les métabolites primaires, les sucres suivants ont été identifiés et dosé : le glucose, le fructose, le saccharose ainsi que des polysaccharides.

Parmi les métabolites secondaires identifiés, les dérivés caféiques sont particulièrement ciblés dans cet extrait notamment l'acide 3,5-dicafeoylquinique et ses isomères ainsi que l'acide chlorogénique et ses isomères. Des flavonoïdes sont également présents en plus faible quantité.

L'extrait alcoolique des parties aériennes fleuries de Tanaisie, *Tanacetum vulgare* objet de la présente invention comprend notamment au moins des sucres, des polysaccharides et des dérivés caféiques.

En particulier, l'extrait alcoolique de Tanaisie, *Tanacetum vulgare* de l'invention comprend :
- 30 à 60% en poids de sucres et de polysaccharides, de préférence 40 à 50% en poids,
- 5 à 20% en poids de dérivés caféiques de préférence 11 à 14% en poids,
les pourcentages étant exprimés en poids, par rapport au poids total de l'extrait sec.

En particulier, l'extrait alcoolique de *Tanaisie, Tanacetum vulgare* de l'invention comprend :
- 15 à 30% en poids de sucres, de préférence 20 à 27% en poids,
- 15 à 30% en poids de polysaccharides, de préférence 17 à 25% en poids, et
- 5 à 20% en poids de dérivés caféiques, de préférence 11 à 14% en poids,
les pourcentages étant exprimés en poids, par rapport au poids total de l'extrait sec.

En particulier, l'extrait alcoolique de Tanaisie, *Tanacetum vulgare* de l'invention comprend :
- 1 à 10% en poids de glucose, de préférence 3 à 8% en poids,
- 5 à 15 % en poids de fructose, de préférence 6 à 12% en poids,
- 5 à 15% en poids de saccharose, de préférence 7 à 10% en poids,
- 15 à 30% en poids de polysaccharides, de préférence 17 à 25% en poids, et
- 5 à 20% en poids de dérivés caféiques, de préférence 11 à 14% en poids,
les pourcentages étant exprimés en poids, par rapport au poids total de l'extrait sec.

En particulier, les sucres comprennent de glucose, de fructose et de saccharose. Avantageusement, ceux-ci sont présents dans les proportions suivantes :
- 1 à 10% en poids de glucose, de préférence 3 à 8% en poids,
- 5 à 15 % en poids de fructose, de préférence 6 à 12% en poids,
- 5 à 15% en poids de saccharose, de préférence 7 à 10% en poids,
les pourcentages étant exprimés en poids, par rapport au poids total de l'extrait sec.

En particulier, l'extrait alcoolique de Tanaisie, *Tanacetum vulgare* de l'invention comprend :
- 30 à 60% en poids de sucres et de polysaccharides, de préférence 40 à 50% en poids,
- 1 à 15% en poids d'acide dicafeoylquinique, de préférence 2 à 8% en poids,
- 1 à 10% en poids d'acide chlorogénique, de préférence 3 à 5% en poids, et
les pourcentages étant exprimés en poids, par rapport au poids sec total de l'extrait.

En particulier, l'extrait alcoolique de Tanaisie, *Tanacetum vulgare* de l'invention comprend :
- 30 à 60% en poids de sucres et de polysaccharides, de préférence 40 à 50% en poids,
- 1 à 10% en poids d'acide 3,5-dicafeoylquinique, de préférence 2 à 6% en poids,
- 0.1 à 5% en poids d'acide 3,4-dicafeoylquinique, de préférence 0.5 à 2% en poids,
- 1 à 10% en poids d'acide chlorogénique, de préférence 3 à 5% en poids, et
les pourcentages étant exprimés en poids, par rapport au poids sec total de l'extrait

En particulier, l'extrait alcoolique sec de *Tanaisie, Tanacetum vulgare* de l'invention comprend en outre entre 0,1 à 10%, de préférence entre 1 et 5% en poids de protéines (dosées selon la méthode bien connue par l'homme du métier Azote*6,25), les pourcentages étant exprimés en poids, par rapport au poids total de l'extrait sec.

Il comprend également des minéraux sous forme de cendres entre 0,1 à 10%, de préférence entre 1 et 5% en poids, les pourcentages étant exprimés en poids, par rapport au poids total de l'extrait sec.

Il comprend aussi une fraction lipophile entre 1 à 10%, de préférence entre 4 et 8% en poids, les pourcentages étant exprimés en poids, par rapport au poids total de l'extrait sec.

Par « dérivés caféiques », on entend l'ensemble des isomères d'acide cafféoylquiniques (l'acide chlorogénique) et dicaffeoylquiniques (l'acide 3,5-dicafeoylquinique et l'acide 3,4-dicafeoylquinique) ayant une fonction acide présents dans l'extrait selon l'invention,.

L'acide 3,5-dicafeoylquinique (I), l'acide 3,4-dicafeoylquinique (II) ou l'acide chlorogénique (III) sont des composés ayant les structures suivantes :

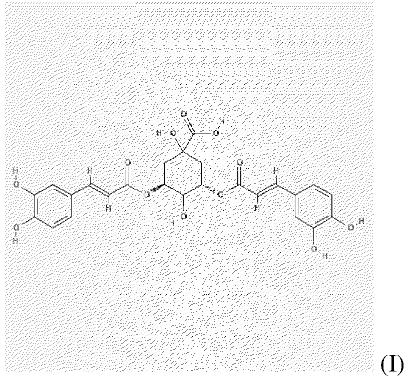

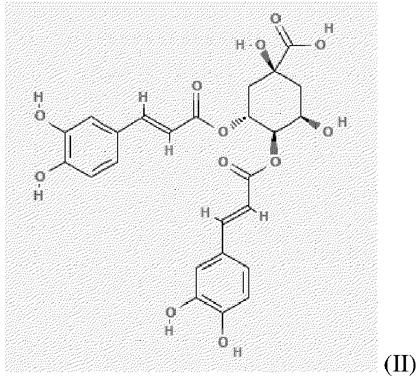

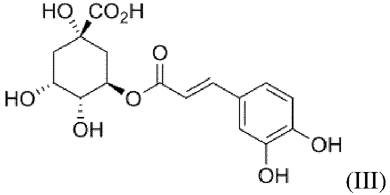

Il est donc du mérite de la demanderesse d'avoir réussi à préparer un extrait de parties aériennes fleuries de Tanaisie, *Tanacetum vulgare* comprenant notamment des sucres, des polysaccharides et des dérivés caféiques, ce qui en fait sa spécificité.

L'extrait des parties aériennes fleuries de Tanaisie, *Tanacetum vulgare* contient diverses familles de métabolites primaires et secondaires.

L'extrait alcoolique de parties aériennes fleuries de Tanaisie, *Tanacetum vulgare* de l'invention peut notamment être obtenu au moyen d'un procédé d'extraction des parties aériennes fleuries de Tanaisie, *Tanacetum vulgare* comprenant les étapes suivantes :
a) le broyage des parties aériennes fleuries de Tanaisie, *Tanacetum vulgare*,
b) au moins une, de préférence au moins deux extractions desdites parties aériennes fleuries broyées en présence d'un premier solvant alcoolique, chaque extraction étant suivie d'une étape de filtration ;
c) le mélange des filtrats liquides issus des extractions de l'étape b) ;
d) filtration du mélange obtenu en c) et, optionnellement, décoloration du filtrat obtenu par adoption sur charbon actif;
e) dilution dans un deuxième solvant alcoolique autre que le premier solvant du mélange obtenu en d), et élimination du premier solvant alcoolique.

De préférence, les parties aériennes fleuries de Tanaisie, *Tanacetum vulgare* mises en oeuvre à l'étape a) sont et sont typiquement choisies parmi les fleurs, les feuilles, les tiges et leurs mélanges.

De préférence, elles sont séchées.

Elles sont notamment broyées ou réduites en morceaux, par exemple à une finesse inférieure à 2 cm, par tout moyen connu de l'homme du métier.

Dans l'étape b), les parties aériennes fleuries de Tanaisie, *Tanacetum vulgare* broyées sont soumises à au moins une, de préférence au moins deux extractions par un ou plusieurs solvants alcooliques, par exemple choisis parmi les monoalcools comprenant de 1 à 4 atomes de carbone (en C₁-C₄), tels que par exemple le méthanol, l'éthanol ou l'isopropanol.

De préférence, le premier solvant alcoolique est un monoalcool comprenant de 2 à 4 atomes de carbone, plus préférentiellement l'éthanol.

De préférence, le premier solvant mono-alcoolique mis en oeuvre en mélange à au moins 90% avec de l'eau, de préférence au moins 95% avec de l'eau.

De préférence, l'éthanol et est mis en oeuvre en mélange à au moins 90% avec de l'eau, de préférence au moins 95% avec de l'eau.

En particulier, l'éthanol est mis en oeuvre en mélange à 96% avec de l'eau.

L'extraction est généralement réalisée en immergeant et en agitant doucement les parties aériennes fleuries de Tanaisie, *Tanacetum vulgare* et broyées dans un ou plusieurs des solvants cités ci-dessus pendant une durée comprise entre 1 et 6 heures, de préférence entre 1 et 3 heures, à une température comprise entre 40°C et 80°C, de préférence entre 50 et 70°C, de préférence encore entre 55 et 65°C.

De préférence, les parties aériennes fleuries de Tanaisie, *Tanacetum vulgare* sont extraites dans le premier solvant alcoolique avec un rapport compris entre 1/20 et 1/5, de préférence entre 0,8/10 et 1,2/10, de préférence de 1/8 en masse de parties aériennes fleuries par rapport à la masse du premier solvant alcoolique.

L'étape d'extraction b) est suivie d'une filtration, par exemple sur un tamis de 100µm, pour éliminer les résidus végétaux.

Selon un mode préféré de réalisation, l'étape b) met en oeuvre au moins deux extractions. Dans ce cas, chaque extraction étant suivie d'une étape de filtration.

A l'issue de la ou des étapes d'extraction b), les filtrats liquides sont récupérés et mélangés.

Le filtrat ou le mélange de filtrats obtenu à l'issue de l'étape c) est alors à nouveau filtré afin d'éliminer les substances insolubles : c'est l'étape d). De préférence, la filtration de l'extrait obtenu en c) est effectuée sur un tamis ou une membrane de filtration de 15 µm. On obtient ainsi un filtrat liquide. Ce filtrat liquide est une fraction alcoolique.

De préférence, entre les étapes d) et e), une étape de décoloration du filtrat obtenu en d) est ajoutée. La décoloration peut se faire par adsorption des pigments présents dans le filtrat sur charbon actif sous agitation pendant une durée comprise entre 1 et 6 heures, de préférence entre 2 et 4 heures, à une température ambiante. De préférence, le charbon actif représente 30% en masse par rapport à la matière sèche d'extrait. Cette étape de décoloration peut être suivie d'une étape de filtration du filtrat décoloré obtenu, sur un tamis ou une membrane de filtration de 4 µm.

L'ordre des étapes de d'élimination du premier solvant et de la dilution du filtrat dans un deuxième solvant est indifférent. En particulier, le solvant présent dans le filtrat liquide peut être éliminé, puis le reste de filtrat est dilué dans un deuxième solvant alcoolique. Alternativement, le filtrat liquide est d'abord dilué dans un deuxième solvant alcoolique, et les solvants sont ensuite éliminés. Encore alternativement, la dilution du filtrat et l'élimination des solvants peut être simultanée.

Le deuxième solvant alcoolique utilisé dans l'étape e) est appelé « autre solvant alcoolique », car il est différent du premier solvant alcoolique utilisé dans l'étape a). Tenant compte de cette limitation, le deuxième solvant alcoolique est typiquement choisi dans le même groupe que celui de l'étape a), i.e. parmi les monoalcools en C₁-C₄ et les diols tels que par exemple le propylène glycol, le 1,3-propanediol ou le dipropylène glycol.

De préférence, la dilution est réalisée dans un diol, de préférence du 1,3-propanediol.

En effet, avantageusement, la dilution de l'étape e) est réalisée dans du 1,3-propanediol avant l'élimination qui se fait par évaporation.

De préférence, l'élimination du premier solvant de l'étape e) se fait par évaporation, notamment à l'aide de l'évaporateur rotatif ou à flux tombant.

L'étape e) prévoit l'étape d'élimination du premier solvant alcoolique utilisé dans l'étape a) de manière à ce que ce dernier soit présent dans une concentration inférieure à 5% dans l'extrait, de préférence inférieure à 1%, de préférence encore inférieure à 0,5%.

L'extrait peut être laissé décanter pendant au moins 6h, de préférence pendant au moins 8h. De préférence encore, l'extrait peut être décanté pendant une durée comprise entre 6 et 18 heures, de préférence entre 6 et 12h. La décantation peut être conduite à une température comprise entre 0 et 25°C, de préférence entre 1 et 10°C, de préférence entre 2 et 5°C.

Ensuite, l'extrait est filtré sur un tamis ou une membrane de filtration, de préférence de 4 µm.

Préférentiellement, l'extrait alcoolique de parties aériennes fleuries de Tanaisie, *Tanacetum vulgare*, selon l'invention est susceptible d'être obtenu par un procédé comprenant les étapes suivantes :
a) le broyage des parties aériennes fleuries de Tanaisie, *Tanacetum vulgare*,
b) au moins une, de préférence au moins deux extractions desdites parties aériennes fleuries broyées en présence d'un premier solvant alcoolique, chaque extraction étant suivie d'une étape de filtration ;
c) le mélange des filtrats liquides issus des extractions de l'étape b) ;
d) filtration du mélange obtenu en c) et, optionnellement, décoloration du filtrat obtenu par adoption sur charbon actif;
e) dilution dans un deuxième solvant alcoolique autre que le premier solvant alcoolique du mélange obtenu en d), et élimination du premier solvant alcoolique.

Avantageusement, l'extrait mis en oeuvre selon l'invention est de couleur claire.

Également, ledit extrait se présente sous une forme suffisamment concentrée pour pouvoir être utilisé sans entraîner les problèmes de formulation habituellement rencontrés aux concentrations nécessaires pour obtenir une activité dans les compositions cosmétiques ou dermatologiques sous forme d'émulsion, et sans présenter une couleur foncée, contrairement aux extraits végétaux obtenus par des procédés usuels, lorsqu'ils sont sous forme concentrée.

En particulier, avant l'évaporation de l'éthanol, l'extrait de parties aériennes fleuries de Tanaisie, *Tanacetum vulgare*, est dilué dans du 1,3-propanediol pour obtenir une composition d'environ 10% en poids d'extrait sec de Tanaisie, *Tanacetum vulgare* et 90% en poids de 1,3-propanediol. L'étape e) prévoit en outre l'étape d'élimination du premier solvant alcoolique utilisé dans l'étape a) de manière à ce que ce dernier soit présent dans une concentration inférieure à 5% dans l'extrait, de préférence inférieure à 1%, de préférence encore inférieure à 0,5%.

De ce fait, l'extrait selon l'invention peut être utilisé directement pour la préparation d'une composition cosmétique.

Selon un autre aspect, l'invention concerne l'utilisation cosmétique d'un extrait alcoolique de parties aériennes fleuries de Tanaisie, *Tanacetum vulgare*, selon l'invention, comme actif apaisant.

Selon un autre aspect, l'invention concerne l'utilisation cosmétique d'un extrait alcoolique de parties aériennes fleuries de Tanaisie, *Tanacetum vulgare*, selon l'invention, pour prévenir et/ou réduire le vieillissement cutané.

En effet, de manière avantageuse, la Demanderesse a trouvé que l'extrait alcoolique de parties aériennes fleuries de Tanaisie, *Tanacetum vulgare* selon l'invention possédait plusieurs activités d'intérêt vis-à-vis de mécanismes physiologiques préventifs ou réparateurs liés au vieillissement cutané ou bien l'inflammation.

La Demanderesse a notamment trouvé, de manière avantageuse, que l'extrait alcoolique de parties aériennes fleuries de Tanaisie, *Tanacetum vulgare* selon l'invention permettait l'expression de CCL2 et CCL5, des molécules faisant partie du SASP5, dans des fibroblastes sénescents. Il permet ainsi de réduire la propagation de la sénescence cutanée et présente donc des propriétés cosmétiques anti-âge.

L'extrait selon l'invention présente des propriétés similaires à celles du rétinol sans les effets indésirables du rétinol. Il permet en effet une augmentation significative de l'épaisseur de l'épiderme, le renforcement de la fonction barrière en augmentant la synthèse de la Loricrine et permet d'augmenter le renouvellement tissulaire.

D'autre part, l'extrait selon l'invention inhibe des voies biologiques comme celle des interférons, des cytokines immunomodulatrices connues pour leur rôle régulateur dans l'inflammation cutanée. Il diminue également l'expression de l'interleukine 33 (IL33) et de CXCL14, respectivement une cytokine et une chemokine, participant également à l'inflammation de la peau. L'extrait joue donc un rôle dans la régulation de l'inflammation cutanée et, par conséquent est reconnue comme ayant des propriétés cosmétiques apaisantes.

Selon encore un autre aspect, l'invention concerne une composition cosmétique comprenant, dans un véhicule cosmétiquement acceptable, un extrait alcoolique de parties aériennes fleuries de Tanaisie, *Tanacetum vulgare* selon les aspects précédents. De préférence, la composition cosmétique est adaptée à une application par voie topique.

De préférence, ledit extrait est présent dans la composition cosmétique ou dermatologique à raison de 0,001 à 10% en poids, par rapport au poids total sec de la composition, en particulier à raison de 0,01 à 10% en poids, de préférence de 0,1 à 5% en poids. Ladite composition cosmétique peut notamment, être adaptée à une application par voie topique.

La composition cosmétique selon l'invention peut se présenter sous la forme d'une composition de soin et/ou de maquillage des matières kératiniques, de préférence du visage.

En particulier, la composition de l'invention peut se présenter sous la forme d'une composition de soin anti-âge de la peau, en particulier du visage.

Selon un autre mode de réalisation, une composition de l'invention peut se présenter sous la forme d'une composition de maquillage du visage, en particulier sous la forme d'un fond de teint, d'une base de teint ou d'une crème teintée.

La composition cosmétique selon l'invention peut comprendre également un solvant choisi en fonction des différents ingrédients et de la forme d'administration.

A titre d'exemples, on peut citer l'eau (de préférence de l'eau déminéralisée ou des eaux florales), un alcool tel que l'éthanol.

Ladite composition cosmétique peut également comprendre, en outre de l'extrait selon l'invention, au moins un additif usuel dans le domaine, tel que par exemple au moins un composé choisi parmi un agent émollient ou humectant, un agent gélifiant et/ou épaississant, un agent tensioactif, une huile, un agent actif, un colorant, un conservateur, un agent antioxydant, un agent actif, une poudre organique ou inorganique, un filtre solaire et un parfum.
- Un ou plusieurs agent(s) humectants, tels que les polyols (glycérine, diglycérine, le propylène glycol, le propanediol, le caprylyl glycol, le pentylène glycol, l'hexanediol), les sucres, les glycosaminoglycanes tels que l'acide hyaluronique et ses sels et esters; et les polyquaterniums

Ledit agent humectant sera présent dans la composition à une teneur de l'ordre de 0.1 à 30%, de préférence 0.005 à 10% en poids total de la composition.
- Un ou plusieurs agent(s) émollient(s) qui peuvent être choisi(s) par exemple parmi les esters tels que les esters de jojoba, les esters d'acides gras et d'alcool gras (octyldodecyl myristate, triethylhexanoin, Dicaprylyl carbonate, Isostearyl isostearate, caprylic/capric triglycéride), les beurres tels que les beurres de karité (butyrospemum parkii butter extract, shea butter ethyl esters) ou de moringa (moringa oil/hydrogenated moringa oil esters), les cires (acacia decurrens flower wax & helianthus annuus cera seed seed wax, C10-18 triglycerides) , les huiles végétales, le phytosqualane, les alcanes (undecane, tridecane)

Ledit agent émollient sera présent dans la composition à une teneur de l'ordre de 0.1 à 30%, de préférence 0.5 à 10% en poids total de la composition.
- Un ou plusieurs agent(s) gélifiants(s) et/ou épaississant(s) de la phase aqueuse, choisi par exemple parmi les dérivés cellulosiques, gommes d'origine végétale (guar, caroube, alginates, carraghenanes, pectines), d'origine microbienne (xanthane), les argiles (laponite), les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique (tel que l'Ammonium AMP/ VP Copolymer, l'Hydroxyethyl Acrylate/ AMPS Copolymer, le Sodium Acrylate/AMPS Copolymer, l'Acrylamide/AMPS Copolymer, l'Acrylates Copolymer, l'Acrylates/C10-30 Alkyl Acrylate Crosspolymer, le Polyacrylate crosspolymer-6, le sodium polyacrylate)

Ledit agent gélifiant et/ou épaississant sera présent dans la composition à une teneur de l'ordre de 0,1 à 10% en poids total de la composition.
- Un ou plusieurs agent(s) tensioactif(s), notamment :
   ^{∗}les tensioactifs anioniques tels que les isethionates, les taurates, les sarcosinates, les glycinates, les glutamates, les phosphates tel que le C20-22 alkyl phosphate
   ^{∗} les tensioactifs amphotères tel que les dérivés de la bétaine, les amphoacétates
   ^{∗} les tensioactifs non ioniques tel que les dérivés de polyglycérol, les dérivés de glucosides (tel que le cetearyl glucoside, le c12-20 alkyl glucoside, l'arachidyl glucoside, le caprylyl/capryl glucoside, le decyl glucoside, le lauryl glucoside), les dérivés de xyloside, les lecithines.

Ledit agent tensioactif, sera présent dans une teneur de l'ordre de 0.1 à 15%, de préférence 0,5 à 10% en poids, par rapport au poids total sec de la composition ;
- Un ou plusieurs agent(s) filmogène(s) tel que les polymères d'origine naturelle, éventuellement modifiées, peuvent être choisis parmi la résine shellac, la gomme de sandaraque, la gomme arabique (ACACIA SENEGAL GUM), les dammars, les élémis, les copals, les polymères cellulosiques, les polymères extraits du fruit de Caesalpinia spinosa et/ou de l'algue Kappaphycus alvarezii, et leurs mélanges.

Ledit agent filmogène, sera présent dans une teneur de l'ordre de 0.1 à 15%, de préférence 0,5 à 10% en poids, par rapport au poids total sec de la composition.
- Un ou plusieurs agent(s) de coloration choisi(s) parmi les pigments, les nacres, les colorants solubles, de préférence solubles dans l'eau. Les pigments peuvent être blancs ou colorés, minéraux et/ou organiques. Les pigments minéraux peuvent être choisis parmi les oxydes de zirconium ou de cérium, ainsi que les oxydes de zinc, de fer, ou de chrome, le dioxyde de titane. Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth.

Ledit agent de coloration, sera présent dans une teneur de l'ordre de 0.01 à 20%, de préférence 2 à 15% en poids, par rapport au poids total sec de la composition.
- Un ou plusieurs agent(s) de charges choisi(s) parmi la lauroyl-lysine, l'amidon, le nitrure de bore et la silice

Ledit agent de charge, sera présent dans une teneur de l'ordre de 0.1 à 10%, de préférence 0,5 à 7% en poids, par rapport au poids total sec de la composition.
- Un ou plusieurs agent(s) actif(s) d'origine naturelle, biotechnologique ou synthétique ayant une activité biologique et ayant une efficacité sur la peau via des sites biologiques, par exemple choisi parmi les vitamines tels que la vitamine C et ses dérivés (ascorbyl glucoside, 3-o-ethyl ascorbic acid, le tétraisopalmitate d'ascorbyle) ,la vitamine A et ses dérivés, la vitamine E et ses dérivés, la vitamine B3 ou Niacinamide, le panthénol, les oligo éléments, l'allantoïne, l'adenosine, les peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5), les extraits végétaux (glycyrrhiza glabra extract, centella asiatica leaf extract, secale cereale seed extract), les extraits de levures, les alpha hydroxyacides tels que l'acide glycolique ou lactique, l'acide tranexamique et ses dérivés tel que le cetyl tranexamique ester etc.

Ledit agent actif sera présent dans la composition à une teneur de l'ordre de 0.1 à 10% en poids total de la composition.

D'autres additifs habituellement utilisés en cosmétique peuvent également être présents dans la composition selon l'invention, notamment des conservateurs, des agents antioxydants ou des parfums bien connus dans le domaine technique.

L'homme du métier est en mesure de choisir, parmi l'ensemble de ces éventuels additifs, aussi bien la nature que la quantité de ceux qui seront ajoutés à la composition, de telle sorte que celle-ci conserve l'ensemble de ses propriétés.

L'invention est illustrée de manière non limitative par les exemples ci-dessous.

### Exemple 1 : Préparation d'un extrait alcoolique des parties aériennes fleuries de Tanaisie, Tanacetum vulgare, selon l'invention

Un extrait alcoolique des parties aériennes fleuries de Tanaisie, *Tanacetum vulgare.*, selon l'invention est préparé par un procédé comprenant les étapes suivantes :
a) le broyage de 1000g des parties aériennes fleuries séchées de *Tanaisie, Tanacetum vulgare,* en poudre
b) Extraction de la poudre dans 10 000g d'éthanol (rapport 1/10 en masse de végétal par rapport à la masse d'éthanol) 96° sous agitation à 60°C pendant 2h ;
   - Tamisage du mélange 1 obtenu à 100µm : le filtrat 1 est mis de côté et les drêches sont récupérées pour une nouvelle extraction;
   - Extraction des drêches dans 10 000g d'éthanol 96° sous agitation à 60°C pendant 2h ;
   - Tamisage du mélange 2 obtenu à 100µm : le filtrat 2 est récupéré et les drêches sont écartées ;
c) Le mélange formé par les filtrats 1 et 2 ;
d) Filtration du surnageant à 15µm ;
   - Décoloration du filtrat obtenu pendant 3h à température ambiante sous agitation ; la décoloration s'effectue par ajout de 30% en masse de charbon actif par rapport à la masse sèche d'extrait (25 g de charbon actif);
   - Filtration sur membranes à 4µm : élimination du charbon actif ;
e) Ajout de 666 g de 1,3-propanediol pour obtenir un mélangé à 10% de matière sèche
   - Evaporation complète de l'éthanol à l'évaporateur rotatif ou à l'évaporateur à flux tombant (concentration finale en éthanol inférieure à 0.5%)
   - Décantation pendant une nuit à 4°C
   - Filtration finale à 4µm.
   740g d'extrait final sont obtenus.

Après analyse, il a été déterminé que l'extrait comprenait :
- 4.7% en poids d'acide chlorogénique et de ses isomères ;
- 5.3% en poids d'acide 3,5-dicafeoylquinique et
- 1.5% en poids d'acide 3,4-dicafeoylquinique.
les pourcentages étant exprimés en poids, par rapport au poids sec total de l'extrait.

### Exemple 2 : Stimulation de l'expression de gènes codant pour des protéines antioxydantes et inhibition de gènes impliqués dans l'inflammation dans des kératinocytes humains normaux traités avec l'extrait selon l'invention.

**Protocole :** Des kératinocytes épidermiques humains normaux issus de trois donneurs différents ont été ensemencés en plaque 24 puits et cultivés en milieu complémenté kératinocyte-SFM (k-SFM) pendant 48h à 37°C et 5% de CO₂. Les cellules ont ensuite été incubées avec ou sans (condition non traitée) 0,025% d'extrait pendant 24h. Chaque condition a été effectuée en duplicat. Les ARN totaux ont été extraits à l'aide de TriPure Isolation Reagent^{®} selon le protocole préconisé par le fournisseur. Les ADN complémentaires ont été synthétisés et un transcriptome a été réalisé sur puce Affymetrix GeneChip Human Transcriptome Array 2.0. L'analyse bioinformatique des gènes dont l'expression est modulée a minima par un facteur 2 a été effectuée avec le logiciel Ingenuity Pathway Analysis (IPA^{®}, QIAGEN).

**Résultats** : Les voies biologiques significativement stimulées par l'extrait de Tanaisie sont majoritairement antioxydantes. Il stimule notamment la voie de réponse au stress oxydatif médiée par NRF2 (nuclear factor erythroid 2-related factor 2, facteur nucléaire 2 lié au facteur érythroïde 2). NRF2 est un facteur de transcription qui permet, une fois activé, d'augmenter l'expression d'enzymes antioxydantes telles que GPX2, NQO1 et TXNRD11. L'extrait de Tanaisie augmente aussi l'expression de SPRR (Small Proline Rich proteins), des protéines connues pour leurs propriétés antioxydantes2. Les gènes impliqués dans la réponse au stress oxydatif ainsi que leur niveau de stimulation par l'extrait de Tanaisie versus témoin non traité (niveau d'expression > 2) sont reportés dans le tableau I.

**Tableau I : Liste des gènes impliqués dans les voies antioxydantes dont l'expression est augmentée par l'extrait de Tanaisie selon l'invention.**

| **Symbole** | **Nom du gène** | **Niveau d'expression (vs témoin non traité)** |
|---|---|---|
| NQO1 | NAD(P)H quinone dehydrogenase 1 | 7,840 |
| SPRR2G | small proline rich protein 2G | 4,800 |
| GPX2 | glutathione peroxidase 2 | 4,040 |
| SPRR1A | small proline rich protein 1A | 2,700 |
| TXNRD1 | thioredoxin reductase 1 | 2,050 |
| SPRR3 | small proline rich protein 3 | 2,030 |

Par sa capacité à stimuler l'expression de différents gènes impliqués dans des voies permettant de limiter le stress oxydatif cellulaire, l'extrait de Tanaisie présente un potentiel cosmétique antioxydant.

D'autre part, comme indiqué dans le tableau II, l'extrait inhibe des voies biologiques comme celle des interférons, des cytokines immunomodulatrices connues pour leur rôle régulateur dans l'inflammation cutanée. Il diminue également l'expression de l'interleukine 33 (IL33) et de CXCL14, respectivement une cytokine et une chemokine, participant également à l'inflammation de la peau.

**Tableau II : Liste des gènes impliqués dans les voies de l'inflammation dont l'expression est diminuée par l'extrait de Tanaisie.**

| Symbole | Nom du gène | Niveau d'expression (vs témoin non traité) |
|---|---|---|
| CXCL14 | C-X-C motif chemokine ligand 14 | -13,6 |
| IFI44L | interferon induced protein 44 like | -5,530 |
| IFIT1 | interferon induced protein with tetratricopeptide repeats 1 | -2,730 |
| IL33 | interleukin 33 | -2,420 |
| IFI44 | interferon induced protein 44 | -2,470 |
| IFIT3 | interferon induced protein with tetratricopeptide repeats 3 | -2,450 |

Par sa capacité à inhiber l'expression de différents gènes impliqués dans l'inflammation cutanée, l'extrait de Tanaisie présente un potentiel cosmétique apaisant.

### Exemple 3 : Diminution de l'expression de gènes impliqués dans le phénotype sécrétoire associé à la sénescence (SASP) dans des fibroblastes humains sénescents traités avec l'extrait selon l'invention.

**Protocole** : Des fibroblastes cutanés humains normaux issus de trois donneurs différents ont été ensemencés en plaque 6 puits et cultivés en milieu DMEM pendant 24h à 37°C et 5% de CO2. Les cellules ont ensuite été stressées par deux traitements à la doxorubicine 100nM pendant 6h pour induire la sénescence. Après 72h de récupération en DMEM + 10% sérum, les fibroblastes devenus sénescents ont été incubées dans du DMEM + 10% de sérum avec ou sans (condition non traitée) 0,0083% ou 0,025% d'extrait de Tanaisie pendant 72h. Les ARN totaux ont ensuite été extraits à l'aide de TriPure Isolation Reagent^{®} selon le protocole préconisé par le fournisseur. Les ADN complémentaires ont été synthétisés et des PCR quantitatives ont été réalisées à l'aide de sondes ciblant CCL2. Un dosage de CCL5 a été réalisé dans le surnageant de culture des cellules à l'aide d'un Multiplex Custom Human G-series Array.

**Résultats** : Les fibroblastes sénescents sécrètent différentes molécules, regroupées sous le nom de SASP pour phénotype sécrétoire associé à la sénescence, favorisant la propagation de la sénescence dans la peau et ainsi son vieillissement.

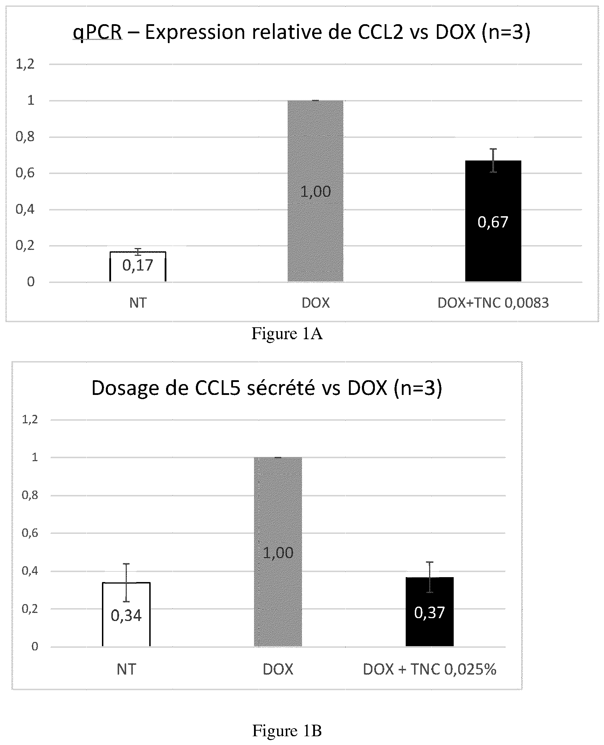

Comme le montre la Figure 1, l'extrait de Tanaisie est capable de réduire l'expression de CCL2 et CCL5, des molécules faisant partie du SASP5, dans des fibroblastes sénescents. Il permet ainsi de réduire la propagation de la sénescence cutanée et présente donc des propriétés cosmétiques anti-âge.

### Exemple 4 : Augmentation de l'épaisseur de l'épiderme pour les explants traités par l'extrait de Tanaisie en formulation

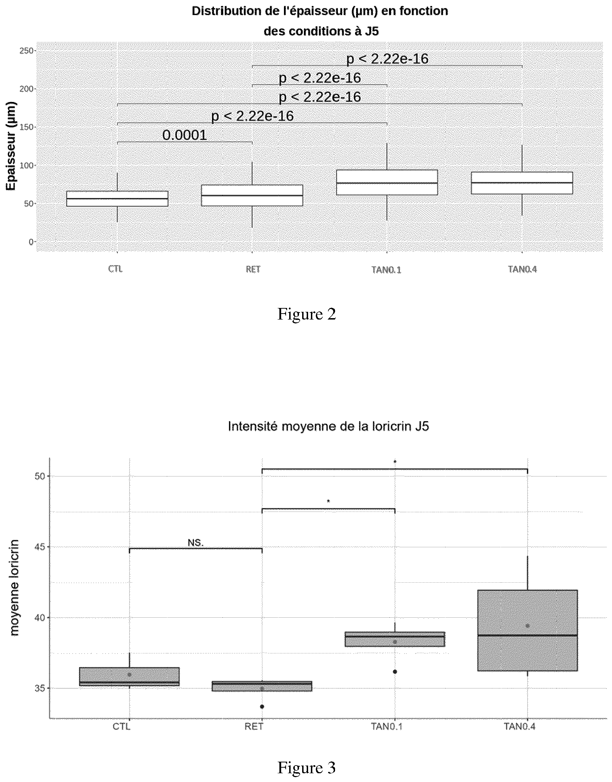

**Protocole** : Avec l'âge l'épaisseur de l'épiderme diminue. Le rétinol est d'ailleurs utilisé et connu pour son impact sur l'épaississement de l'épiderme. L'extrait de Tanaisie a été testé sur explants de peau afin d'évaluer son impact sur l'épaisseur de l'épiderme en comparaison au rétinol. Les explants sont des disques de peau d'un cm de diamètre issus de plastie humaine normale. Pour cette étude, la plastie est issue d'une femme caucasienne de 35 ans et de phototype 2 à 3. Les explants sont maintenus en survie, dans un milieu de culture développé au laboratoire Ex-Vivo, pendant 5 jours à 37°C et 5% de CO₂. Durant cette période, une partie des explants appelés « CTL» (indiqué comme CTL dans la Figure 2) n'ont reçu aucun traitement alors qu'une autre partie a été traitée avec une formule simplex contenant l'extrait de Tanaisie à 0,1% (indiqué comme TAN 0,1 dans la Figure 2) ou à 0,4% (indiqué comme TAN 0,4) versus le rétinol 0,05% (indiqué comme RET dans la Figure 2). Chaque condition a été testée deux fois. Les pourcentages sont exprimés en poids, par rapport au poids total de l'extrait sec.

**Résultats** : L'application de l'extrait de Tanaisie permet une augmentation significative de l'épaisseur de l'épiderme (Figure 2). L'utilisation de l'extrait de Tanaisie, par rapport au contrôle, permet une augmentation de l'épaisseur de l'épiderme de 36,9% (l'extrait de Tanaisie à 0,1%) ou encore de 37,4% (TAN0,4) contre 7,7% obtenue avec le rétinol, comme indiqué dans le Tableau III ci-après. La distribution de l'épaisseur a été mesurée après 5 jours.

Comparé au rétinol, l'extrait de Tanaisie est bien plus performant permettant un bénéfice de 27,1% ou 27,6% d'augmentation d'épaisseur exprimée en µm obtenue respectivement avec TAN0,1 et TAN0,4.

Par son effet sur l'épaississement de l'épiderme l'extrait de Tanaisie présente donc des propriétés cosmétiques anti-âge.

Dans la Figure 2, la valeur de « p » correspond à la significativité entre deux conditions avec un test de Wilcoxon.

Le test de Wilcoxon donne la probabilité "p" pour que deux lots soient significativement différents. La différence entre deux lots n'est pas significative si p>0,1 (indiquée comme n.s, dans le tableau). La différence entre deux lots est significative si 0.1>p>0.05, soit une probabilité entre 90-95% pour que deux lots soient significativement différents ou 0.01<p<0.05, soit une probabilité entre 95-99% pour que deux lots soient significativement différents ou p<0. 01, donc une probabilité de plus de 99% pour que deux lots soient significativement différents, ou p<0.001, donc une probabilité de plus de 99,9% pour que deux lots soient significativement différents.

Dans la Figure 2, les valeurs de p sont de 0,0001 pour les lots CTL/RET0.05 et inférieures à 2,22e⁻¹⁶ pour les autres lots.

**Tableau III : Mesure de l'augmentation de l'épaisseur de l'épiderme par l'extrait de Tanaisie.**

| Condition après 5 jours (J5) | Mesure épiderme* (µm) | % augmentation versus contrôle CTL (sans produit) |
|---|---|---|
| CTL | 56.0 | Référence |
| RET | 60.3 | +7.7 |
| TAN0.1 | 76.7 | +36.9 |
| TAN0.4 | 77 | +37.4 |
| | * médiane | |

| Condition après 5 jours (J5) | | % augmentation versus RET |
|---|---|---|
| RET | | Référence |
| CTL | | -7.1 |
| TAN0.1 | | +27.1 |
| TAN0.4 | | +27.6 |

### Exemple 5 : Renforcement de la fonction barrière des explants traités à l'extrait de Tanaisie

**Protocole** : Le rétinol est connu pour son impact sur l'épaississement de l'épiderme, cependant en contrepartie il induit une inflammation au niveau de la peau. L'exemple 4 a démontré que l'extrait de Tanaisie permettait un meilleur épaississement de l'épiderme. L'exemple 5 a pour but d'évaluer l'impact de l'extrait de Tanaisie sur le renforcement de la fonction barrière versus l'utilisation du rétinol. Ce renforcement sera évalué par quantification d'un immunomarquage de la loricrine. Cette technique permet de révéler une protéine spécifique sur une coupe de tissu via l'utilisation d'un anticorps spécifique de la protéine d'intérêt. Un second anticorps couplé à un fluorochrome permettra la détection du premier anticorps à l'aide d'un microscope à fluorescence. La fonction principale de la protéine loricrine est de renforcer l'enveloppe cellulaire cornifiée et d'améliorer sa fonction de barrière défensive. Par analogie, plus la loricrine est exprimée plus la fonction barrière est renforcée.

Les explants, issus d'une plastie caucasienne de 35ans, ont été maintenus en culture pendant 5 jours à 37°C et 5% de CO₂. Comme pour l'exemple 4, une partie des explants n'a subi aucun traitement (indiqué comme CTL dans la Figure 3), et une partie a été traitée par application de l'extrait de Tanaisie à 0,1% (indiqué comme TAN0,1 dans la Figure 3) ou à 0,4% (indiqué comme TAN0,4 dans la Figure 3) ou du rétinol à 0,05% (indiqué comme RET dans la Figure 3) en formule. Chaque condition a été testée deux fois. Les pourcentages sont exprimés en poids, par rapport au poids total de l'extrait sec.

**Résultats** : Comme illustré par le graphe de la Figure 3, les explants traités par l'extrait de Tanaisie présentent une augmentation significative de la synthèse de la Loricrine par rapport aux explants traités avec le rétinol ce qui implique une fonction barrière renforcée.

En effet, les explants traités avec l'extrait de Tanaisie à 0,1% et 0,4% synthétisent respectivement 9,4%* et 12,7%* de plus de loricrine que les explants traités avec le rétinol , comme indiqué dans le Tableau IV ci-après. Il y a également un renforcement obtenu par rapport au contrôle même si la significativité n'est pas atteinte.

L'exemple 4 a permis de constater que l'extrait de Tanaisie avait un effet plus impactant que le rétinol sur l'épaississement de l'épiderme. L'exemple 5 permet de démontrer que contrairement aux effets irritants du rétinol sur la peau l'extrait de Tanaisie permet de renforcer la fonction barrière. Cet extrait présente donc des propriétés cosmétiques anti-âge similaires à celles présentées par le rétinol sans les inconvénients du rétinol.

Dans la Figure 3, la valeur de « p » correspond à la significativité entre deux conditions avec un test de Wilcoxon; comme indiqué dans le cas de la Figure 2 ci-dessus.

Dans la Figure 3, les valeurs de p sont NS pour les lots CTL/RET et 0.01<p<0.05 (*, valeur de p dans la Figure 3) pour les autres lots, soit une probabilité entre 95-99% pour que deux lots soient significativement différents.

**Tableau IV : Mesure de la synthèse de loricrine dans les explants.**

| | | Croissance en % | |
|---|---|---|---|
| Condition J5 | Référence J5 | Moyenne _loricrin | |
| RET | CTL | -2.70 | |
| TAN0.1 | CTL | 6.40 | |
| TAN0.4 | CTL | 9.6 | |
| CTL | RET | 2.8 | |
| TAN0.1 | RET | 9.4 | |
| TAN0.4 | RET | 12.70 | |

| RET_J5 | TAN0.1_J5 | TAN0.4_J5 | Condition |
|---|---|---|---|
| 0.86 | 0.11 | 0.23 | CTL_J5 |
| | 0.029 | 0.029 | RET_J5 |
| | | 0.89 | TAN0.1_J5 |

### Exemple 6 : Renouvellement cellulaire des explants traités à l'extrait de Tanaisie

**Protocole** : Le rétinol est connu pour stimuler le renouvellement cellulaire. L'exemple 4 a démontré que l'extrait de Tanaisie permettait un meilleur épaississement de l'épiderme et l'exemple 5 - un renforcement de la fonction barrière. L'exemple 6a pour but d'évaluer l'impact de l'extrait de Tanaisie sur le renouvellement cellulaire versus l'utilisation du rétinol. Ce renouvellement sera évalué par quantification d'un immunomarquage de Ki67. Cette technique permet de révéler une protéine spécifique sur une coupe de tissu via l'utilisation d'un anticorps spécifique de la protéine d'intérêt. Un second anticorps couplé à un fluorochrome permettra la détection du premier anticorps à l'aide d'un microscope à fluorescence. La protéine Ki67 est un marqueur de prolifération. Par analogie, plus Ki67 est exprimé plus le renouvellement tissulaire est important.

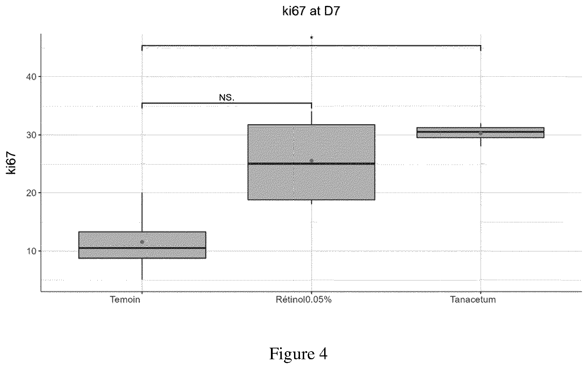

Les explants, issus d'une plastie caucasienne de 61 ans, ont été maintenus en culture 7 jours à 37°C et 5% de CO₂. Comme pour l'exemple 4, une partie des explants n'a subi aucun traitement (indiqué comme Témoin dans la Figure 4), et une partie a été traitée par application de l'extrait de Tanaisie à 0,4% (indiqué comme Tanacetum dans la Figure 4) ou du rétinol à 0,05% (indiqué comme Rétinol0.05% dans la Figure 4) en formule duplicats. Chaque condition a été testée deux fois. Les pourcentages sont exprimés en poids, par rapport au poids total de l'extrait sec.

**Résultats** : Comme illustré par le graphe de la Figure 4, l'extrait de Tanaisie permet par rapport au contrôle de stimuler le renouvellement tissulaire des explants en augmentant le nombre de noyaux marqués positivement par Ki67 (+163%* vs CTL) après 7 jours de traitement (indigué « D7 » dans la Figure 4), comme indiqué dans le Tableau V ci-après. En comparant les explants traités à l'extrait de Tanaisie par rapport aux explants traités avec le rétinol, la synthèse de Ki67 est également augmentée de 18.6% (n.s).

L'exemple 4 a permis de constater que l'extrait de Tanaisie avait un effet plus impactant que le rétinol sur l'épaississement de l'épiderme. L'exemple 5 permet de démontrer que contrairement aux effets irritants du rétinol sur la peau l'extrait de Tanaisie permet de renforcer la fonction barrière. Et enfin l'exemple 6 confirme que l'extrait de Tanaisie permet d'augmenter le renouvellement tissulaire. Ces trois exemples permettent de confirmer que l'extrait de Tanaisie présente donc des propriétés cosmétiques anti-âge.

Dans la Figure 4, la valeur de « p » correspond à la significativité entre deux conditions avec un test de Wilcoxon; comme indiqué dans le cas de la Figure 2 ci-dessus.

Dans la Figure 4, les valeurs de p sont NS pour les lots Temoin/ Rétinol0.05% et 0.01<p<0.05 (*, valeur de p dans la Figure 4) pour l'autre lot Temoin/ Tancetum, soit une probabilité entre 95-99% pour que deux lots soient significativement différents.

**Tableau V : Mesure de la synthèse de loricrine dans les explants.**

| | | Croissance en % |
|---|---|---|
| Condition J7 | Référence J7 | Ki67 |
| Rétinol0.05% | Témoin | 121.70 |
| Tanacetum | Témoin | 163.00 |
| Condition J7 | Référence J7 | Ki67 |
| Témoin | Rétinol | -54.90 |
| | 0.05% | |
| Tanacetum | Rétinol | 18.6 |
| | 0.05% | |

| Rétinol0.05% _ après 7 jours (D7) | Tanacetum après 7 jours (D7) | Condition |
|---|---|---|
| 0.11 | 0.029 | Témoin après 7 jours (D7) |
| | 0.77 | Rétinol0.05%_après 7 jours (D7) |

### Exemple 7: compositions cosmétiques

Les compositions suivantes peuvent être préparées de façon classique pour l'homme du métier. Les quantités indiquées ci-dessous sont exprimées en pourcentages pondéraux. Les ingrédients en majuscules sont identifiés conformément à la dénomination INCI.

### A - émulsion huile/eau fluide

| **Nom INCI** | **(% W/W)** |
|---|---|
| Limnanthes alba (meadowfoam) seed oil | 1-10 |
| Camellia oleifera seed oil | 1-10 |
| Caprylic/capric triglycéride | 1-10 |
| Dicaprylyl carbonate | 1-5 |
| Squalane | 1-10 |
| Ammonium acryloyldimethyltaurate/VP copolymer | 0.1-5 |
| Hydroxyethyl acrylate/sodium acryloyldimethyl taurate copolymer & sorbitan isostearate & polysorbate 60 | 0.1-2 |
| Xanthan gum | 0.01-5 |
| Biosaccharide gum-1 | 0.01-5 |
| Sodium hyaluronate | 0.01-3 |
| Glycerin | 1-30 |
| Polyquaternium-51 | 1-10 |
| Tocopheryl acetate | 0.1-5 |
| Niacinamide | 0.1-5 |
| Polianthes tuberosa | 0.01-5 |
| Sodium pca | 0.5-5 |
| Saccharide isomerate | 0.5-5 |
| Extrait des parties aériennes fleuries de Tanaisie selon l'invention | 0.001-10 |
| Yeast extract | 0.1-5 |
| CI 77891 (titanium dioxide) & aluminum hydroxide & magnésium chloride & sodium lauroyl glutamate & lysine | 1-10 |
| CI 77891 (titanium dioxide) & mica & tin oxide | 1-10 |
| Kappaphycus alvarezii Extract & Caesalpinia spinosa Fruit Extract | 0.1-5 |
| Titanium dioxide & stearic acid & alumina | 1-7 |
| Phenylbenzimidazole sulfonic acid | 1-3 |
| Glycols (Caprylyl Glycol and/or Pentylene Glycol and/or Butylène Glycol and/or propanediol) | 0,1-10 |
| Water | Qs 100 |

### b - émulsion huile/eau crème

| **Nom INCI** | **(% w/w)** |
|---|---|
| Jojoba esters | 1-5 |
| Limnanthes alba (meadowfoam) seed oil | 0.1-5 |
| Canola oil | 1-10 |
| Argania spinosa kernel oil | 0.1-10 |
| Moringa oil/hydrogenated moringa oil esters | 1-10 |
| C8-12 acid triglycéride | 1-5 |
| Lauroyl lysine | 1-5 |
| Camellia oleifera seed oil | 1-10 |
| Squalane | 1-10 |
| Ammonium acryloyldimethyltaurate/vp copolymer | 1-5 |
| Cetearyl alcohol & cetearyl glucoside | 1-7 |
| Sodium lauroyl glutamate | 0.1-1 |
| Hydrogenated lecithin | 0.1-5 |
| Chondrus crispus (carrageenan) | 0.1-5 |
| Sclerotium gum | 0,01-2 |
| Centella asiatica leaf extract | 0.1-5 |
| Adenosine | 0.1-0.5 |
| Niacinamide | 0.1-5 |
| Secale cereale (rye) seed extract | 0.1-5 |
| Palmitoyl tripeptide-1 & palmitoyl tetrapeptide-7 | 1-5 |
| Plankton extract | 0.1-5 |
| Yeast extract | 1-3 |
| Extrait des parties aériennes fleuries de Tanaisie selon l'invention | 0.001-10 |
| Glycyrrhiza glabra extract | 0.001-5 |
| Tranexamic cetyl ester | 0.001-5 |
| Ascorbyl glucoside | 0.001-5 |
| Water | Qs 100 |

Ces compositions peuvent être appliquées tous les jours, matin et/ou soir, sur la peau.

## Revendications

1. Extrait alcoolique des parties aériennes fleuries de Tanaisie, *Tanacetum vulgare*, comprenant au moins des sucres, des polysaccharides et des dérivés caféiques.

2. Extrait alcoolique des parties aériennes fleuries de Tanaisie, *Tanacetum vulgare* selon la revendication 1, comprenant :
∘ 30 à 60% en poids de sucres et de polysaccharides, de préférence 40 à 50% en poids,
∘ 5 à 20% en poids de dérivés caféiques, de préférence 6 à 14% en poids,
les pourcentages étant exprimés en poids, par rapport au poids total sec de l'extrait.

3. Extrait alcoolique des parties aériennes fleuries de Tanaisie, *Tanacetum vulgare* selon l'une quelconque des revendications précédentes, dans lequel les sucres comprennent :
∘ 1 à 10% en poids de glucose, de préférence 3 à 8% en poids,
∘ 5 à 15 % en poids de fructose, de préférence 6 à 12% en poids,
∘ 5 à 15% en poids de saccharose, de préférence 7 à 10% en poids,
les pourcentages étant exprimés en poids, par rapport au poids total sec de l'extrait.

4. Extrait alcoolique des parties aériennes fleuries de de Tanaisie, *Tanacetum vulgare* selon l'une quelconque des revendications précédentes, comprenant
- 30 à 60% en poids de sucres et de polysaccharides, de préférence 40 à 50% en poids,
- 1 à 15% en poids d'acide dicafeoylquinique, de préférence 2 à 8% en poids,
- 1 à 10% en poids d'acide chlorogénique, de préférence 3 à 5% en poids, et
les pourcentages étant exprimés en poids, par rapport au poids sec total de l'extrait.

5. Extrait alcoolique des parties aériennes fleuries de Tanaisie, *Tanacetum vulgare* selon l'une quelconque des revendications précédentes, comprenant
- 30 à 60% en poids de sucres et de polysaccharides, de préférence 40 à 50% en poids,
- 1 à 10% en poids d'acide 3,5-dicafeoylquinique, de préférence 2 à 6% en poids,
- 0.1 à 5% en poids d'acide 3,4-dicafeoylquinique, de préférence 0.5 à 2% en poids,
- 1 à 10% en poids d'acide chlorogénique, de préférence 3 à 5% en poids, et
les pourcentages étant exprimés en poids, par rapport au poids sec total de l'extrait.

6. Procédé d'extraction des parties aériennes fleuries de Tanaisie, *Tanacetum vulgare.*, comprenant les étapes suivantes :
a) le broyage des parties aériennes fleuries de Tanaisie, *Tanacetum vulgare,*
b) au moins une, de préférence au moins deux extractions desdites parties aériennes fleuries séchées broyées en présence d'un premier solvant alcoolique, chaque extraction étant suivie d'une étape de filtration ;
c) le mélange des filtrats liquides issus des extractions de l'étape b) ;
d) filtration du mélange obtenu en c) et, optionnellement, décoloration du filtrat obtenu par adoption sur charbon actif;
e) dilution dans un deuxième solvant alcoolique autre que le premier solvant alcoolique du mélange obtenu en d), et élimination du premier solvant alcoolique.

7. Procédé selon la revendication 6, **caractérisé en ce que** les parties aériennes fleuries de Tanaisie, *Tanacetum vulgare* mises en oeuvre à l'étape a) sont des parties aériennes séchées choisies parmi les fleurs, les feuilles, les tiges et leurs mélanges.

8. Procédé selon l'une quelconque des revendications 6 ou 7, **caractérisé en ce qu'**à l'étape a), les parties aériennes fleuries de Tanaisie, *Tanacetum vulgare* sont broyées à une finesse inférieure à 2 cm et/ou **en ce que** le premier solvant alcoolique de l'étape b) est un monoalcool comprenant de 1 à 4 atomes de carbone, de préférence l'éthanol.

9. Procédé selon l'une quelconque des revendications 6 à 8, **caractérisé en ce que** les extractions de l'étape b) sont réalisées pendant une durée comprise entre 1 et 6 heures, de préférence entre 1 et 3 heures, à une température comprise entre 40°C et 80°C, de préférence entre 50 et 70°C et/ou **en ce que** chaque extraction de l'étape b) est suivie d'une filtration sur un tamis de 100µm.

10. Procédé selon l'une quelconque des revendications 6 à 9, **caractérisé en ce que** la filtration de l'extrait obtenu en c) mise en oeuvre à l'étape d) est effectuée sur un tamis ou une membrane de filtration de 4µm et/ou **en ce que**, entre les étapes d) et e), est ajoutée une étape de décoloration du filtrat obtenu en d), de préférence par adsorption sur charbon actif, suivie d'une étape de filtration du filtrat décoloré obtenu, de préférence sur un tamis ou une membrane de filtration de 4µm.

11. Procédé selon l'une quelconque des revendications 6 à 10, **caractérisé en ce que** l'élimination de l'étape e) se fait par évaporation, puis la dilution est réalisée dans du 1,3-propanediol.

12. Extrait alcoolique de parties aériennes fleuries de Tanaisie, *Tanacetum vulgare,* **caractérisé en ce qu'**il est susceptible d'être obtenu par un procédé selon l'une quelconque des revendications 6 à 11.

13. Utilisation cosmétique d'un extrait alcoolique de parties aériennes fleuries de Tanaisie, *Tanacetum vulgare* selon l'une quelconque des revendications 1 à 3, comme actif apaisant.

14. Utilisation cosmétique d'un extrait alcoolique de parties aériennes fleuries de Tanaisie, *Tanacetum vulgare* selon l'une quelconque des revendications 1 à 3, pour prévenir et/ou réduire le vieillissement cutané.

15. Composition cosmétique comprenant, dans un véhicule cosmétiquement acceptable, un extrait alcoolique de parties aériennes fleuries de Tanaisie, *Tanacetum vulgare* selon l'une quelconque des revendications 1 à 3.

16. Composition cosmétique selon la revendication 16 **caractérisée en ce qu'**elle est adaptée à une application par voie topique.
